(19) Europäisches Patentamt
European Patent Office
Office européen des brevets

(11) Veröffentlichungsnummer: **0 037 996**
**B1**

# (12) EUROPÄISCHE PATENTSCHRIFT

(45) Veröffentlichungstag der Patentschrift: **10.11.82**

(51) Int. Cl.³: **C 07 C 135/02,** B 01 F 17/22, C 09 K 7/02

(21) Anmeldenummer: **81102558.4**

(22) Anmeldetag: **04.04.81**

(54) **Verfahren zur Herstellung von Aminoxiden und deren Verwendung als Tenside.**

(30) Priorität: **16.04.80 DE 3014510**

(43) Veröffentlichungstag der Anmeldung: **21.10.81 Patentblatt 81/42**

(45) Bekanntmachung des Hinweises auf die Patenterteilung: **10.11.82 Patentblatt 82/45**

(84) Benannte Vertragsstaaten: **AT BE FR GB IT NL**

(56) Entgegenhaltungen:
**BE-A-626 505**
**DE-A-2 532 469**

**KIRK-OTHMER «Encyclopedia of Chemical Technology» Band 2, 3. Auflage 1978, J. WILEY AND SONS, New York, Chichester, Brisbane, Toronto, Seite 265**

(73) Patentinhaber: **Th. Goldschmidt AG, Goldschmidtstrasse 100, D-4300 Essen (DE)**

(72) Erfinder: **Wagner, Helmut, Bachstrasse 2, D-6834 Ketsch (DE)**

EP 0 037 996 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Aminoxiden und deren Verwendung als Tenside

Die Erfindung betrifft ein Verfahren zur Herstellung von Aminoxiden der allgemeinen Formel

$$\begin{array}{l} \qquad\quad R^3 \\ \qquad\quad | \\ R^1NHR^2N \rightarrow O \\ \qquad\quad | \\ \qquad\quad R^4 \end{array} \qquad I$$

In dieser allgemeinen Formel haben die Substituenten folgende Bedeutung

$R^1$ ist der von den Naphthensäuren hergeleitete Naphthenoylrest. Dabei sind unter Naphthensäuren im Sinne vorliegender Erfindung die aus den Rohölen und deren Produkten durch Extraktion mit Lauge und nachfolgendem Ansäuern erhaltenen natürlichen Säuren zu verstehen. Es handelt sich dabei um Säuregemische, in denen neben geradkettigen Carbonsäuren insbesondere alkylierte Cyclopentan- und Cyclohexancarbonsäuren überwiegen. Nähere Angaben über die Zusammensetzung von Naphthensäuren können dem «Erdöl-Lexikon» von Dr. Alfred Hüthig, Verlag Heidelberg, Seite 192, entnommen werden. Für die Herstellung der erfindungsgemäss zu verwendenden Aminoxide sind besonders die Naphthensäuren geeignet, die eine Säurezahl von 80 bis 350, vorzugsweise 120 bis 250, aufweisen.

$R^2$ ist ein Alkylenrest mit 2 bis 5 Kohlenstoffatomen. Der Alkylenrest ist bevorzugt geradkettig. Besonders bevorzugt ist der Äthylen- oder Propylenrest.

Die Substituenten $R^3$ und $R^4$ sind gleich oder verschieden. Sie haben die Bedeutung eines Methyl- oder Äthylrestes. Besonders bevorzugt ist dabei der Methylrest.

Die Herstellung dieser Verbindung gelingt erfindungsgemäss dadurch, dass man Amine der allgemeinen Formel

$$R^1NHR^2NR^3R^4 \qquad II$$

in Form einer wässrigen, feinteiligen Dispersion bei erhöhter Temperatur mit sauerstoffabspaltenden Verbindungen in an sich bekannter Weise oxidiert.

Es hat sich gezeigt, dass die Verwendung einer wässrigen, feinteiligen Dispersion der Amine es gestattet, die Reaktionszeit, die für die Oxidation benötigt wird, stark abzukürzen. Während bei groben Verteilungen der Amine im Wasser, z.B. mittels eines Rührers, Oxidationszeiten von mehreren Stunden erforderlich sind, gelingt die Herstellung der Aminoxide aus disperser Phase bereits in einem Zeitraum von 30 bis 60 Minuten. Die Verwendung einer wässrigen, feinteiligen Dispersion ist dabei gegenüber der Verwendung eines Hilfslösungsmittels vorteilhafter, da bei Mitverwendung eines Lösungsmittels besondere Vorsichtsmassnahmen zu beachten sind und aus Kostengründen das Lösungsmittel rückgewonnen werden muss.

Als sauerstoffabspaltende Verbindungen werden entsprechend dem Stand der Technik Wasserstoffsuperoxid, Ozon, Alkalihypochlorit oder anorganische oder organische Persäuren verwendet. Besonders bevorzugt ist die Verwendung von Wasserstoffsuperoxid. Zum Stand der Technik wird auf das Buch «Tenside, Textilhilfsmittel, Waschrohstoffe», Band 1, von Kurt Lindner, Wissenschaftliche Verlagsgesellschaft, Hugenwald, und auf Houben-Weyl, «Methoden der organischen Chemie», Band Stickstoffverbindungen II/III, Seite 190 ff., verwiesen.

Die Oxidation wird vorzugsweise bei Temperaturen von 70°C und darüber, insbesondere bei Temperaturen von 80 bis 95°C, durchgeführt.

Die Dispersionen der Amine der allgemeinen Formel II sind vorzugsweise 20 bis 40 gewichtsprozentig. Besonders bevorzugt sind 20 bis 30 gewichtsprozentige Dispersionen.

Die feinteiligen Dispersionen können mit Hilfe einer hochtourigen, scherkraftreichen Dispergiervorrichtung erhalten werden. Es hat sich gezeigt, dass man die erhaltenen Dispersionen durch einen geringen Zusatz von Aminoxiden der allgemeinen Formel I stabilisieren kann. Eine besondere Ausführungsform des erfindungsgemässen Verfahrens ist deshalb dadurch gekennzeichnet, dass man wässrige Dispersionen der Amine der Formel II verwendet, welche 0,5 bis 5 Gew.-%, insbesondere 0,5 bis 2 Gew.-%, Aminoxid der allgemeinen Formel I (bezogen auf Gesamtdispersion) enthalten.

Bei der bevorzugten Oxidation der Amine mit Wasserstoffperoxid entstehen keine störenden Reaktionsnebenprodukte.

Im Verlauf der Oxidation wandelt sich die zu Beginn der Reaktion eingesetzte Dispersion des Amins in eine klare bis schwach opaleszierende Lösung des Aminoxids der Formel I um. Bei Verbindungen, deren Naphthenoylrest relativ hochmolekular ist, können auch trübe Lösungen entstehen, die jedoch durch geringen Zusatz einer insbesondere organischen Säure in eine klare Lösung überführt werden können. Je nach Gehalt der Lösungen an Aminoxiden weisen die Lösungen unterschiedliche Viskosität auf. Die Lösungen können dünnflüssig, mittelviskos bis gelartig sein.

Die Aminoxide werden als oberflächenaktive Substanzen meist in Form einer wässrigen Lösung verwendet, so dass es sich erübrigt, aus der restlichen Lösung die Aminoxide in fester Form herzustellen. Es können die erhaltenen Lösungen bzw. Verdünnungen hiervon eingesetzt werden. Es ist jedoch auch möglich, durch eine schonende Destillation, insbesondere unter Verwendung eines Dünnschichtverdampfers, das Aminoxid in praktisch wasserfreie Form zu überführen.

Die erfindungsgemäss hergestellten Aminoxide zeigen hervorragende oberflächenaktive Eigenschaften. Sie können zur Emulgierung von Erdöl, Erdölderivaten oder bituminösen Produkten, z.B. zur Herstellung von Bitumenemulsionen für den Strassenbau, verwendet werden. Aufgrund des Naphthenoylrestes haben die erfindungsgemäss hergestellten Aminoxide eine besondere Affinität zu Erdöl und Erdölprodukten. Sie ermöglichen die Herstellung sehr stabiler Erdöl-Wasseremulsionen. Sie sind gegen einen Salzgehalt des Wassers und Temperaturen ≦100°C weitgehend unempfindlich. Sie erniedrigen bereits in sehr geringen Konzentrationen die Grenzflächenspannung zwischen Erdöl und Wasser, z.B. zwischen Rohöl und Lagerstättenwasser. Die erfindungsgemäss hergestellten Aminoxide sind deshalb insbesondere zur Erhöhung der Ausbeute von Erdöllagerstätten, z.B. als Hilfsstoffe für die tertiäre Erdölförderung, geeignet. Es ist mit Hilfe der erfindungsgemäss hergestellten Verbindungen möglich, das in der Lagerstätte nach den konventionellen Massnahmen verbliebene Erdöl zu aktivieren und freizusetzen und für die Förderung zu mobilisieren. Die erfindungsgemäss hergestellen Verbindungen sind mit anderen anionaktiven, kationaktiven oder nichtionogenen Tensiden sowie mit den üblicherweise verwendeten organischen Verdickungsmitteln verträglich.

Aus der DE-OS 25 32 469 ist die Verwendung von Carboxybetainen mit einer Naphthenoylgruppe für die Erdölgewinnung bekannt. In der DE-OS 27 36 408 ist die Herstellung entsprechender Sulfobetaine zu diesem Zweck beschrieben. Die erfindungsgemäss hergestellten Aminoxide haben gegenüber diesen Verbindungen eine Reihe von vorteilhaften Eigenschaften. Eine dieser Eigenschaften besteht darin, dass eine starke Erniedrigung der Grenzflächenspannung bereits bei niederen Temperaturen, z.B. 20 bis 40°C, beobachtet wird, während die Betaine der vorgenannten Art die stärkste Erniedrigung der Oberflächenspannungswerte bei Temperaturen von 50°C und mehr aufweisen. Da die Temperatur der Lagerstätten sehr unterschiedlich ist, ist es möglich geworden, mit der Substanzgruppe der Aminoxide, welche einen Naphthenoylrest aufweisen, auch solche Lagerstätten zu behandeln, die eine niedrige Lagerstättentemperatur aufweisen.

Das erfindungsgemässe Verfahren sowie die Eigenschaften der nach diesem Verfahren hergestellten Verbindungen werden anhand der folgenden Beispiele noch näher erläutert.

Beispiel 1

Herstellung von 1-Naphthenoylamino-2-diäthylaminoxid-äthan

$$\begin{array}{l} \qquad\qquad\quad C_2H_5 \\ \qquad\qquad\quad | \\ RNHCH_2CH_2N \rightarrow O \\ \qquad\qquad\quad | \\ \qquad\qquad\quad C_2H_5 \end{array}$$

R ist der Naphthenoylrest einer Naphthensäure, die durch folgende Kennzahlen charakterisiert ist:

| | |
|---|---|
| Säurezahl | 205 |
| Molgewicht | 273 |
| Neutralölanteil | 8,7 Gew.-% |
| Brechungsindex $n_{19}$ | 1,4835 |
| Dichte $d_4^{23}$ | 0,961 |

185,5 g eines Aminoamids, das durch Umsetzung der Naphthensäure mit 2-Amino-1-diäthylamino-äthan hergestellt worden ist, werden unter intensivem Rühren in Wasser von 70°C eingetragen, dem vorher 1,94 g des herzustellenden Aminoxids in Form einer 30%igen wässrigen Lösung zugesetzt worden waren. Die Dispersion wird anschliessend mit einem Dispergiergerät homogenisiert. Es wird ein im Handel unter dem Namen «Ultra Turrax» erhältliches Gerät verwendet. Die Homogenisierung erfolgt bei 20 000 U/min während einer Dauer von 2 min. Die Tröpfchengrösse der dispergierten Phase beträgt ≦30 µm. Die Temperatur der Dispersion wird nun auf 85°C erhöht. Der Dispersion werden innerhalb von 30 min 85 g 30%iges Wasserstoffperoxid unter Rühren zugefügt. Dabei steigt die Temperatur an. Das Reaktionsgemisch wird abgekühlt, damit die Temperatur 98°C nicht überschreitet. Nach weiteren 30 min ist die Reaktion weitgehend beendet. Durch Zugabe von 34 g 96%iger Essigsäure wird ein pH-Wert von ca. 5,5 eingestellt.

Eine 30%ige wässrige Lösung des Verfahrensproduktes ist bei Raumtemperatur homogen und dünnflüssig und besitzt einen Brechungsindex von $n_{20}$ 1,3875. Umsetzungsgrad > 90%.

Beispiel 2

Herstellung von 1-Naphthenoylamino-2-diäthylaminoxid-äthan

$$\begin{array}{l} \qquad\qquad\quad C_2H_5 \\ \qquad\qquad\quad | \\ RNHCH_2CH_2N \rightarrow O \\ \qquad\qquad\quad | \\ \qquad\qquad\quad C_2H_5 \end{array}$$

R ist der Naphthenoylrest einer Naphthensäure, die durch folgende Kennzahlen charakterisiert ist:

| | |
|---|---|
| Säurezahl | 121 |
| Molgewicht | 496 |
| Neutralölanteil | 12,8 Gew.-% |
| Brechungsindex $n_{19}$ | 1,5153 |
| Dichte $d_4^{23}$ | 0,986 |

149 g eines Aminoamids, das durch Umsetzung der Naphthensäure mit 2-Amino-1-diäthylamino-äthan hergestellt worden ist, werden unter intensivem Rühren in Wasser von 70°C eingetragen, dem vorher 9,15 g des herzustellenden Aminoxids in Form einer 30%igen wässrigen Lösung zugesetzt worden waren, und entsprechend Beispiel 1 homogenisiert. Die Temperatur der Dispersion wird nun auf 85°C erhöht. Der Dispersion werden innerhalb von 30 min 141,7 g 30%iges

Wasserstoffperoxid unter Rühren zugefügt. Dabei steigt die Temperatur an. Das Reaktionsgemisch wird gekühlt, damit die Temperatur 98°C nicht überschreitet. Nach weiteren 30 min wird die Reaktion beendet. Durch Zugabe von 17 g 96%iger Essigsäure wird ein pH-Wert von ca. 5,5 eingestellt.

Die erhaltene Lösung ist bei Raumtemperatur dickflüssig und zeigt beim Rühren Schlierenbildung. Der Brechungsindex beträgt $n_{20}$ 1,3931. Umsetzungsgrad > 90%.

Beispiel 3

Herstellung von 1-Naphthenoylamino-2-dimethylaminoxid-äthan

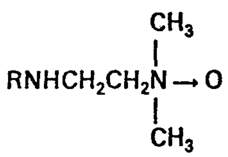

R ist der Naphthenoylrest einer Naphthensäure, die durch folgende Kennzahlen charakterisiert ist:

| | |
|---|---|
| Säurezahl | 121 |
| Molgewicht | 496 |
| Neutralölanteil | 12,8 Gew.-% |
| Brechungsindex $n_{19}$ | 1,5153 |
| Dichte $d_4^{23}$ | 0,986 |

141,5 g eines Aminoamids, das durch Umsetzung der Naphthensäure mit 2-Amino-1-dimethylamino-äthan hergestellt worden ist, werden in 213,8 g Wasser von 70°C gegeben. Dem Wasser sind vorher 4,85 g des herzustellenden Aminoxids in Form der 30%igen wässrigen Lösung zugesetzt worden. Die Dispersion wird entsprechend Beispiel 1 homogenisiert. Die Temperatur der Dispersion wird nun auf 85°C erhöht. Der Dispersion werden innerhalb von 60 min 141,7 g 30%iges Wasserstoffperoxid unter Rühren zugefügt. Dabei steigt die Temperatur an. Das Reaktionsgemisch wird gekühlt, damit die Temperatur 98°C nicht überschreitet. Nach ca. 35 min ist die Reaktion beendet. Der pH-Wert des Verfahrensproduktes wird durch Zugabe von 17 g 96%iger Essigsäure auf etwa 5,5 eingestellt.

Die 30%ige wässrige Lösung des Verfahrensproduktes ist bei Raumtemperatur sehr dickflüssig und hat einen Brechungsindex von $n_{20}$ 1,3902. Umsetzungsgrad > 90%.

Beispiel 4

Herstellung von 1-Naphthenoylamino-3-dimethylaminoxid-propan

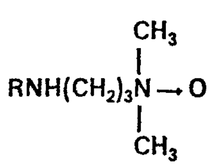

R ist der Naphthenoylrest einer Naphthensäure, die durch folgende Kennzahlen charakterisiert ist:

| | |
|---|---|
| Säurezahl | 184 |
| Molgewicht | 296 |
| Neutralölanteil | 9,3 Gew.-% |
| Brechungsindex $n_{19}$ | 1,4840 |
| Dichte $d_4^{23}$ | 0,952 |

190 g eines Aminoamids, das durch Umsetzung der Naphthensäure mit Dimethylaminopropylamin hergestellt worden ist, werden in 355g Wasser von 70°C eingetragen. Die Homogenisierung erfolgt gemäss Beispiel 1. Die Temperatur der Dispersion wird nun auf 85°C erhöht. Der Dispersion werden innerhalb von 15 min 85 g 30%iges Wasserstoffperoxid unter Rühren zugefügt. Dabei steigt die Temperatur an. Das Reaktionsgemisch wird gekühlt, damit die Temperatur 98°C nicht überschreitet. Die Reaktion ist nach 35 min beendet. Das Verfahrensprodukt wird durch Zugabe von 34 g 96%iger Essigsäure auf einen pH-Wert von ca. 5 eingestellt.

Die 30%ige wässrige Lösung ist bei Raumtemperatur dünnflüssig. Der Brechungsindex beträgt $n_{20}$ 1,3871. Umsetzungsgrad > 90%.

Beispiel 5

Herstellung von 1-Naphthenoylamino-4-diäthylaminoxid-butan

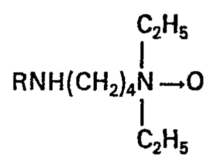

R ist der Naphthenoylrest einer Naphthensäure, die durch folgende Kennzahlen charakterisiert ist:

| | |
|---|---|
| Säurezahl | 184 |
| Molgewicht | 296 |
| Neutralölanteil | 9,3 Gew.-% |
| Brechungsindex $n_{19}$ | 1,4840 |
| Dichte $d_4^{23}$ | 0,952 |

211 g eines Aminoamids, das durch Umsetzung der Naphthensäure mit 4-Diäthylaminobutylamin hergestellt worden ist, werden in 435 g Wasser von 70°C eingetragen und analog Beispiel 1 homogenisiert. Zur zusätzlichen Stabilisierung der Dispersion werden 22 g einer 30%igen wässrigen Lösung des herzustellenden Aminoxids zugefügt. Die Temperatur der Dispersion wird nun auf 85°C erhöht. Der Dispersion werden innerhalb von 30 min 62,3 g 30%iges Wasserstoffperoxid unter Rühren zugefügt. Dabei steigt die Temperatur an. Das Reaktionsgemisch wird gekühlt, damit die Temperatur 98°C nicht überschreitet. Die Reaktion ist nach 45 min beendet. Das Verfahrensprodukt wird durch Zugabe von 33 g 96%iger Essigsäure auf einen pH-Wert von ca. 5,5 eingestellt.

Die 30%ige wässrige Lösung des erhaltenen Aminoxids ist bei Raumtemperatur dünnflüssig.

Ihr Brechungsindex beträgt $n_{20}$ 1,3877. Umsetzungsgrad > 90%.

Beispiel 6

Herstellung von 1-Naphthenoylamino-1-methyl-4-diäthylaminoxid-pentan

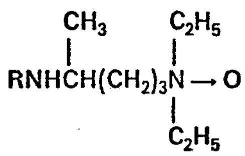

R ist der Naphthenoylrest einer Naphthensäure, die durch folgende Kennzahlen charakterisiert ist:

| | |
|---|---|
| Säurezahl | 184 |
| Molgewicht | 296 |
| Neutralölanteil | 9,3 Gew.-% |
| Brechungsindex $n_{19}$ | 1,4840 |
| Dichte $d_4^{23}$ | 0,952 |

109,1 g eines Aminoamids, das durch Umsetzung der Naphthensäure mit 1-Diäthylamino-4-aminopentan hergestellt worden ist, werden in Wasser von 70°C eingetragen, dem vorher 7,5 g des herzustellenden Aminoxids in Form einer 30%igen wässrigen Lösung zugesetzt worden waren. Die Dispersion wird entsprechend Beispiel 1 homogenisiert. Die Temperatur der Dispersion wird nun auf 85°C erhöht. Der Dispersion werden innerhalb von 20 min 42,5 g 30%iges Wasserstoffperoxid unter Rühren zugefügt. Dabei steigt die Temperatur an. Das Reaktionsgemisch wird gekühlt, damit die Temperatur 98°C nicht überschreitet. Nach weiteren 40 min wird die Reaktion beendet. Durch Zugabe von 15,6 g 96%iger Essigsäure wird ein pH-Wert von ca. 5,5 eingestellt.

Die 30%ige wässrige Lösung des Verfahrensproduktes ist bei Raumtemperatur dünnflüssig. Der Brechungsindex beträgt $n_{20}$ 1,3951. Umsetzungsgrad > 90%.

Anwendungstechnische Prüfungen

Die Messung der Grenzflächenspannungen Rohöl/synthetisches Lagerstättenwasser erfolgte mit dem sogenannten «Spinning Drop»-Gerät als Funktion der Temperatur. Die Messtechnik der «Spinning Drop»-Methode beruht auf der Ausmessung eines Öltropfens, der in einer spezifisch schwereren Tensidlösung schwebt, die in einer Kapillare eingeschlossen ist, welche horizontal angebracht ist und mit bestimmter Drehzahl rotiert. Diese «Spinning Drop»-Methode ist Bestandteil der «API Recommended Practices for Laboratory, Testing of Surface Active Agents for Well Stimulation» des American Petroleum Institute, Washington, D.C.

Messreihe A

Das synthetische Lagerstättenwasser entspricht DIN 50 900 mit folgenden Kenndaten:

| | |
|---|---|
| Dichte bei 19,4°C | 1,0049 g/ml |
| pH bei 20°C | 7,1 |
| NaCl | 2,725 Gew.-% |
| $MgSO_4 \cdot 7\,H_2O$ | 0,681 Gew.-% |
| $MgCl_2 \cdot 6\,H_2O$ | 0,486 Gew.-% |
| $CaCl_2 \cdot 6\,H_2O$ | 0,233 Gew.-% |
| $NaHCO_3$ | 0,019 Gew.-% |
| Salzgehalt | 4,144 Gew.-% |

Die Kenndaten des für die Mesung verwendeten Rohöls betragen:

| | |
|---|---|
| Dichte bei 20°C | 0,8800 g/ml |
| Dichte bei 80°C | 0,8488 g/ml |
| Brechungsindex $n_{20}$ | 1,5005 |
| Anilinpunkt | 90,5°C |
| Kinematische Viskosität | |
| bei 20°C | $6{,}0 \cdot 10^1 mm^2 \cdot s^{-1}$ |
| bei 60°C | $1{,}93 \cdot 10^1 mm^2 \cdot s^{-1}$ |

Es wird die Grenzflächenspannung von Rohöl gegen das genannte synthetische Lagerstättenwasser bestimmt, wobei in dem Lagerstättenwasser 1000 ppm 1-Naphthenoylamino-2-diäthylaminoxid-äthan, hergestellt nach Beispiel 2, gelöst sind. Es werden folgende Grenzflächenspannungen in Abhängigkeit von der Temperatur bestimmt:

| Temperatur °C | Grenzflächenspannung in mN/m |
|---|---|
| 40 | $1{,}5 \cdot 10^{-4}$ |
| 50 | $1{,}3 \cdot 10^{-2}$ |
| 60 | $4{,}7 \cdot 10^{-4}$ |
| 70 | $2{,}15 \cdot 10^{-2}$ |
| 80 | $5{,}0 \cdot 10^{-2}$ |

Messreihe B

1-Naphthenoylamino-3-dimethylaminoxid-propan wird entsprechend Beispiel 4 mit verschiedenen Naphthensäuren unterschiedlichen Molekulargewichtes und damit unterschiedlicher Hydrophobie hergestellt. Die erhaltenen Aminoxide haben ein Molgewicht zwischen 370 und 407. Sie sind um so hydrophober, je höher das Molekulargewicht ist. Mit steigendem Molekulargewicht sinkt somit der HLB-Wert. (Der HLB-Wert ist ein direktes Mass der Hydrophilie. Seine Bestimmung ist in dem Aufsatz von W. C. Griffin «Classification of surface-active agents by HLB» in J. Soc. Cosmetic Chemists 1, 311 (1950) näher erläutert.) Damit verändert sich die stoffliche Verteilung des Tensidwirkstoffes zwischen den Phasen und die Art des Grenzflächenzustandes.

Es werden wiederum die Grenzflächenspannungen von Rohöl gegen synthetisches Lagerstättenwasser in der vorher beschriebenen Weise bestimmt, wobei das gleiche Rohöl und das gleiche synthetische Lagerstättenwasser verwendet werden.

Alle Grenzflächenspannungswerte in mN/m

| Temperatur °C | Molgewichte 370 | 378 | 384 | 394 | 399 | 407 |
|---|---|---|---|---|---|---|
| 30 | $5,0 \cdot 10^{-4}$ | $3,5 \cdot 10^{-5}$ | $2,8 \cdot 10^{-3}$ | $1,2 \cdot 10^{-2}$ | $2,8 \cdot 10^{-4}$ | $1,45 \cdot 10^{-2}$ |
| 40 | $1,2 \cdot 10^{-4}$ | $2,1 \cdot 10^{-4}$ | $7,0 \cdot 10^{-5}$ | $3,4 \cdot 10^{-3}$ | $4,7 \cdot 10^{-5}$ | $8,5 \cdot 10^{-2}$ |
| 50 | $1,5 \cdot 10^{-4}$ | $1,7 \cdot 10^{-4}$ | $1,1 \cdot 10^{-3}$ | $1,8 \cdot 10^{-4}$ | $7,0 \cdot 10^{-5}$ | $2,2 \cdot 10^{-2}$ |
| 60 | $1,0 \cdot 10^{-3}$ | $1,6 \cdot 10^{-3}$ | $2,7 \cdot 10^{-3}$ | $2,0 \cdot 10^{-3}$ | $2,6 \cdot 10^{-4}$ | $4,0 \cdot 10^{-2}$ |
| 70 | $1,3 \cdot 10^{-2}$ | $1,2 \cdot 10^{-2}$ | $2,8 \cdot 10^{-2}$ | $3,8 \cdot 10^{-2}$ | $1,25 \cdot 10^{-2}$ | $4,2 \cdot 10^{-2}$ |
| 80 | $2,0 \cdot 10^{-2}$ | $2,2 \cdot 10^{-2}$ | $3,9 \cdot 10^{-2}$ | $6,2 \cdot 10^{-2}$ | $4,0 \cdot 10^{-2}$ | $5,5 \cdot 10^{-2}$ |

Messreihe C

Als Tensid wird 1-Naphthenoylamino-3-dimethylaminoxid-propan eines Molekulargewichtes 378 verwendet. Es wird in einer Menge von 1000 ppm in einem synthetischen Lagerstättenwasser folgender Zusammensetzung gelöst:

| | mg/l | Gew.-% |
|---|---|---|
| NaCl | 165.000 | 14,73 |
| $CaCl_2 \cdot 6\,H_2O$ | 49.349 | 4,41 |
| $MgCl_2 \cdot 6\,H_2O$ | 12.810 | 1,14 |
| KCl | 750 | 0,07 |
| KBr | 400 | 0,03 |
| KJ | 20 | - |
| LiCl | 100 | 0,01 |
| $NH_4Cl$ | 350 | 0,03 |
| $SrCl_2 \cdot 6\,H_2O$ | 1.681 | 0,15 |
| $BaCl_2 \cdot 2\,H_2O$ | 58 | - |
| $NaHCO_3$ | 650 | 0,06 |
| $Na_2SO_4 \cdot 10\,H_2O$ | 680 | 0,06 |
| $NaBO_2 \cdot 4\,H_2O$ | 523 | 0,05 |
| $FeSO_4 \cdot 7\,H_2O$ | 366 | 0,03 |
| | 232.738 | 20,77 |

Dichte bei 18,6°C: 1,1213 g/ml
pH: 6,8

Es wird das gleiche Rohöl wie in den vorherigen Messreihen verwendet:

| Temperatur °C | Grenzflächenspannung in mN/m |
|---|---|
| 30 | $2,0 \cdot 10^{-4}$ |
| 40 | $1,6 \cdot 10^{-2}$ |
| 50 | $4,7 \cdot 10^{-2}$ |
| 60 | $3,6 \cdot 10^{-2}$ |
| 70 | $3,4 \cdot 10^{-2}$ |
| 80 | $7,2 \cdot 10^{-2}$ |

Messreihe D

Die Messung erfolgt wie bei den vorherigen Messreihen mit synthetischem Lagerstättenwasser nach DIN 50 900 mit dem bereits beschriebenen Rohöl. Als Tensid wird das nach Beispiel 5 hergestellte 1-Naphthenoylamino-4-diäthylaminoxid-butan verwendet. 1000 ppm sind hiervon in dem synthetischen Lagerstättenwasser gelöst. Die Grenzflächenspannung wird in Abhängigkeit von der Temperatur wie folgt ermittelt:

| Temperatur °C | Grenzflächenspannung in mN/m |
|---|---|
| 35 | $1,6 \cdot 10^{-4}$ |
| 40 | $< \cdot 10^{-5}$ |
| 50 | $3,0 \cdot 10^{-3}$ |
| 60 | $2,8 \cdot 10^{-2}$ |
| 70 | $8,0 \cdot 10^{-2}$ |

Die erzielte Erniedrigung der Grenzflächenspannung zwischen Rohöl und Tensid enthaltendem Lagerstättenwasser hängt von der stofflichen Verteilung des Tensidwirkstoffes zwischen Öl- und Wasserphase ab. Diese Verteilung wird einerseits durch den HLB-Wert bestimmt und ist andererseits temperaturabhängig. Hieraus ergibt sich vermutlich der unstetige Kurvenverlauf.

Vergleich der erfindungsgemäss verwendeten Verbindungen mit Betainen gemäss DE-PS 25 32 469

Die Vergleichsmessungen werden entsprechend den vorgenannten anwendungstechnischen Prüfungen durchgeführt. Es wird das synthetische Lagerstättenwasser gemäss DIN 50 900 verwendet. Als Rohöl wird das Rohöl der Messreihe A eingesetzt. Das Vergleichsprodukt hat folgende Formel

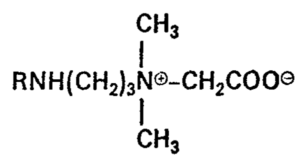

R = Naphthenoylrest einer Naphthensäure

Dieses Betain kann am besten mit der Verbindung, die in der Messreihe B eingesetzt worden ist, verglichen werden. Das Molgewicht des Betains beträgt etwa 530.

Das Betain wird in einer Menge von 1000 ppm in dem synthetischen Lagerstättenwasser gelöst. Die nach der «Spinning Drop»-Methode gemessenen Grenzflächenspannungswerte zeigen folgende Grösse und Abhängigkeit von der Temperatur:

| Temperatur °C | Grenzflächenspannung in mN/m |
|---|---|
| 30 | $3{,}2 \cdot 10^{-2}$ |
| 40 | $2{,}2 \cdot 10^{-2}$ |
| 50 | $1{,}9 \cdot 10^{-2}$ |
| 60 | $1{,}4 \cdot 10^{-2}$ |
| 70 | $1{,}1 \cdot 10^{-2}$ |
| 80 | $2{,}8 \cdot 10^{-2}$ |

Die Grenzflächenspannungswerte zeigen gegenüber den erfindungsgemäss zu verwendenden Verbindungen kein Temperaturminimum. Die erfindungsgemäss zu verwendenden Verbindungen weisen innerhalb ihrer Minima Grenzflächenspannungswerte auf, die etwa zwei Zehnerpotenzen niedriger liegen, als sie bei den Carboxybetainen ermittelt werden können.

**Patentansprüche**

1. Verfahren zur Herstellung von Aminoxiden der allgemeinen Formel

$$R^1NHR^2\overset{\overset{\displaystyle R^3}{|}}{\underset{\underset{\displaystyle R^4}{|}}{N}}\rightarrow O$$

wobei $R^1$ der Naphthenoylrest,
$R^2$ ein Alkylenrest mit 2 bis 5 Kohlenstoffatomen,
$R^3$, $R^4$ gleich oder verschieden und ein Methyl- oder Äthylrest sind,
dadurch gekennzeichnet, dass man Amine der allgemeinen Formel

$R^1NHR^2NR^3R^4$

in Form einer wässrigen, feinteiligen Dispersion bei erhöhter Temperatur mit sauerstoffabspaltenden Verbindungen in an sich bekannter Weise oxidiert.

2. Verfahren nach Anspruch 1, dadurch gekennzeichnet, dass man die Oxidation bei Temperaturen ≧ 70°C durchführt.

3. Verfahren nach Anspruch 1 oder 2, dadurch gekennzeichnet, dass man das Amin in Form einer 20 bis 40 gewichtsprozentigen Dispersion oxidiert.

4. Verfahren nach einem oder mehreren der vorhergehenden Ansprüche, dadurch gekennzeichnet, dass man das Verfahren mit einer Amindispersion durchführt, welche 0,5 bis 5 Gew.-%, bezogen auf Dispersion, Aminoxid der allgemeinen Formel

$$R^1NHR^2\overset{\overset{\displaystyle R^3}{|}}{\underset{\underset{\displaystyle R^4}{|}}{N}}\rightarrow O$$

enthält.

5. Verwendung der nach einem oder mehreren der Ansprüche 1 bis 3 hergestellten Aminoxide als Tenside für die tertiäre Erdölförderung.

**Claims**

1. Process for the preparation of amine oxides of the general formula

$$R^1NHR^2\overset{\overset{\displaystyle R^3}{|}}{\underset{\underset{\displaystyle R^4}{|}}{N}}\rightarrow O$$

wherein $R^1$ is a naphthenoyl radical, $R^2$ is an alkylene radical having 2 to 5 carbon atoms, and $R^3$ and $R^4$ are identical or different and are a methyl or ethyl radical, characterised in that amines of the general formula

$R^1NHR^2NR^3R^4$

in the form of an aqueous, finely particulate dispersion are oxidised in the manner known per se with oxygen-releasing compounds at an elevated temperature.

2. Process according to Claim 1, characterised in that the oxidation is carried out at temperatures ≧ 70°C.

3. Process according to Claim 1 or 2, characterised in that the amine is oxidised in the form of a 20 to 40 per cent by weight dispersion.

4. Process according to one or more of the preceding claims, characterised in that the process is carried out with an amine dispersion which, relative to the dispersion, contains 0.5 to 5% by weight of an amine oxide of the general formula

$$R^1NHR^2\overset{\overset{\displaystyle R^3}{|}}{\underset{\underset{\displaystyle R^4}{|}}{N}}\rightarrow O$$

5. Use of the amine oxides prepared according to one or more of Claims 1 to 3 as surfactants for tertiary petroleum production.

**Revendications**

1. Procédé de fabrication d'aminoxydes de formule générale

$$R^1NHR^2\overset{\overset{\displaystyle R^3}{|}}{\underset{\underset{\displaystyle R^4}{|}}{N}}\rightarrow O$$

où $R^1$ est un reste naphténoyle
$R^2$ un reste alcoyle avec 2 à 5 atomes de carbone
$R^3$, $R^4$ identiques ou différents, sont un reste mé-

thyle ou éthyle,
caractérisé en ce que l'on oxyde des amines de la formule générale

$R^1NHR^2NR^3R^4$

sous forme d'une dispersion aqueuse à particules fines à température élevée par des composés libérant de l'oxygène de façon connue en soi.

2. Procédé selon la revendication 1, caractérisé en ce que l'on effectue l'oxydation à des températures $\geqq 70°C$.

3. Procédé selon la revendication 1 ou 2, caractérisé en ce que l'on oxyde l'amine sous forme d'une dispersion à 20 jusqu'à 40 pourcent en poids.

4. Procédé selon une ou plusieurs des revendications précédentes, caractérisé en ce que l'on effectue le procédé avec une dispersion d'amine qui contient 0,5 à 5% en poids par rapport à la dispersion d'une aminoxyde de la formule générale

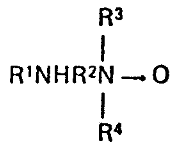

5. Utilisation des aminoxydes préparés selon une ou plusieurs des revendications 1 à 3 comme substances tensioactives pour l'exploitation tertiaire du pétrole.